# EUROPEAN PATENT SPECIFICATION

(11) **EP 3 399 932 B1**
(45) Date of publication and mention of the grant of the patent: **03.04.2024**
(21) Application number: 17701040.2
(22) Date of filing: 07.01.2017
(51) Int. Cl.: A61B 18/14, A61B 34/20

(54) **SYSTEM FOR POSE CONTROLLED ABLATION**
SYSTEM ZUR POSITIONS- UND ORIENTIERUNGSGESTEUERTEN ABLATION
SYSTÈME POUR ABLATION COMMANDÉE PAR POSE

(30) Priority: 07.01.2016 EP 16150502
(43) Date of publication of application: 14.11.2018
(73) Proprietor: Universität Bern, 3012 Bern (CH)
(72) Inventor: WEBER, Stefan, 3067 Boll (CH); CANDINAS, Daniel, 3006 Bern (CH); TINGUELY, Pascale, 3004 Bern (CH)
(74) Representative: Schulz Junghans Patentanwälte PartGmbB
(86) International application number: PCT/EP2017/050290
(87) International publication number: WO 2017/118750

(56) References cited:
- WO-A1-2015/121096
- US-A- 5 840 025
- US-A1- 2008 300 588
- US-A1- 2011 251 607
- US-A1- 2015 196 351

## Description

The invention relates to a system for creating an ablation volume as well as a method for controlling the amount of energy emitted per time by a head of an applicator for creating an ablation volume within a target object.

Ablation refers to the medical process of indirect removal of organs and/or parts thereof by destruction through introduction of any type of energy into the body. More specifically, in oncology tumors are destroyed by introducing energy (i.e. heat) or cold into the tumor tissue, causing the tumor tissue to be destroyed. To date, medical ablations are typically carried out using needle like applicators that are manually or robotically placed in or near an anatomical target and where the placement process is supported by medical imaging and if available instrument guidance technology. US2008/0300588 A1 discloses an ablation system according to the prior art. Based on the above the problem underlying the present invention is to provide a system for creating an ablation volume that allows a more precise and reproducible creation of an ablation volume with respect to the target object.

This problem is solved by a system having the features of claim 1. Preferred embodiments are stated in the sub claims and are described below.

According to claim 1 said system for creating an ablation volume comprising a target object or at least a portion thereof, comprises:
- an applicator comprising a head, which applicator is configured to be inserted into the patient's body so as to position said head into or close to a target object (e.g. a tumor) located inside said body, and wherein the applicator is configured to emit via said head energy provided to the applicator so as to create an ablation volume comprising at least a region of said target object,
- a tracking system configured to track the pose of the head with respect to the pose of the target object, wherein the tracking system is further configured to provide a signal indicative of the relative pose of the head of the applicator with respect to the target object, and
- an energy control unit configured to control emission of said energy by the applicator in response to said signal.

Particularly, in the framework of the present invention, the notion pose refers to the position and the orientation of the respective body and therefore particularly comprises at least six parameters, namely three parameters for characterizing the position of the respective body, and three parameters for characterizing the orientation of the respective body.

Preferably, said applicator is formed as an elongated needle to allow for a percutaneous access to the body of the patient.

Using the system according to the present invention, ablation volumes can be created that are dependent in their properties (e.g. position, orientation, size and shape) from the spatial relationships between the applicator head and the target object.

This allows to control the correct creation of ablation volumes with respect to a target structure with better precision. Further, irregular shaped ablation volumes might be created that allow the complete ablation of irregular shaped target objects with the best available sensitivity and specificity.

According to a preferred embodiment of the present invention, the energy control unit is configured to automatically trigger emission of energy via the head of said applicator towards the target object for generating said ablation volume when the distance between the head and the target object as measured by the tracking system falls below a pre-defined value.

Further, according to a preferred embodiment of the present invention, the tracking system is configured to determine the pose of the head of the applicator and the pose of the target object using at least one of: electromagnetic tracking, optical tracking, optical-fiber based tracking, time-of-flight-based tracking, or any combination thereof.

Particularly, in electromagnetic tracking, a transmitter (magnetic field generator) is preferably used to induce a current in sensor coils that can be embedded into the tracked objects. Further, in optical tracking, a stereo camera is preferably used to track fiducial markers that are attached to the instrument or anatomical structure of interest. Furthermore, in optical-fiber-based tracking, the bending of a fiber optics is measured through fibre bragg gates (FBG) to estimate the location and orientation along its length. Finally, in time-of-flight based tracking, a range imaging camera system may be used that resolves distance based on the known speed of light, measuring the time-of-flight of a light signal between the camera and the subject for each point of the image.

Further, in an embodiment of the present invention, the tracking means/system comprise(s) a display means (e.g. a display for displaying information optically) for displaying the position or pose of the head of the applicator with respect to the position or pose of the target object, preferably in real-time.

Further, according to a preferred embodiment of the present invention, the energy control unit is configured to dynamically control the temporal and spatial delivery of energy to the target object via the head of the applicator using said signal which is particularly provided by the tracking system in a quasi-continuous fashion. Thus the energy control unit controls the emission of energy depending on said relative pose of the head of the applicator with respect to the target. Further, temporal control particularly means that the emission of energy is a distinct function of time.

Further, according to the invention, the energy control unit is configured to control emission of energy, such that the energy that is delivered per unit time and unit of volume to the target object is a linear (particularly continuous) function derived from the relative pose (i.e. 6D) between the head and the target object and/or from a derivative of said relative pose (i.e. a speed, an acceleration etc.). Particularly, said delivered energy may increase and/or decrease depending on the distance between the head and the target object.

Particularly, said delivered energy may increase and/or decrease depending on the position of the head with respect to the target object.

Further, particularly, said delivered energy may increase and/or decrease depending on the position of the head and on the spatial orientation of the applicator or of the head with respect to the target object (i.e. depending on the relative pose).

Further, according to a preferred embodiment of the present invention, said energy which the applicator is configured to emit via its head causes the target object to heat up within said ablation volume so that subsequently material properties of the target object in the ablation volume are changed. Particularly, said energy is one of: energy in the form of alternating electromagnetic fields (i.e. radiofrequency, microwaves, light) or other sorts of energy.

Further, according to a preferred embodiment of the present invention, the applicator comprises a handle for manually moving the applicator or a means for automatically moving the applicator. Using a means for moving the applicator automatically eases creation of an arbitrarily shaped ablation volume, particularly according to a preexisting image-based plan.

Further, according to a preferred embodiment of the present invention, the system is configured to generate an ablation volume of a pre-defined shape by moving the applicator along a pre-defined track by means of the means for automatically moving the applicator.

Further, according to a preferred embodiment of the present invention, the tracking system comprises a first tracking device for measuring the pose of the target object, wherein said first tracking device is configured to be placed into the body of a patient comprising said target object inside said body, and wherein preferably the first tracking device is a first transmitter configured for transmitting an electromagnetic signal indicative of the position or pose of the target object. However, alternatively said first tracking device is an externally attached first tracking device, i.e., is arranged outside the patient.

Further, according to a preferred embodiment of the present invention, the tracking system comprises a second tracking device for measuring the pose of the applicator head, wherein said second tracking device is arranged on the applicator, particularly on the head of the applicator, and wherein preferably the second tracking device is a second transmitter configured for transmitting an electromagnetic signal indicative of the position or pose of the head of the applicator.

Further, according to a preferred embodiment of the present invention, the system comprises an elongated flexible means (or an elongated flexible device) comprising said first tracking device at a tip of said flexible means (or device), wherein said flexible means (or device) is configured to be placed into or close to the target object via a body lumen of the patient, particularly via the arterial or venous vascular system of the patient.

Preferably, according to a preferred embodiment of the present invention, said elongated flexible means or device is a intravascular catheter, preferably configured for transcatheter arterial chemoembolization (also denoted as TACE), or a guide wire for a catheter (e.g. of a catheter configured for TACE).

Here, particularly said catheter or guide wire comprising said first tracking device in the form of an electromagnetic transmitter is guided as close as possible to the target object (e.g. a tumor). Using a subtraction angiography, the pose of the target object with respect to the first tracking device can be inferred and a spatial target position can be defined. The head of the applicator comprises the second tracking device which also is an electromagnetic transmitter. The relative position or pose of the head of the applicator with respect to the guide wire or catheter (second tracking device) is continuously measured and may be displayed on a display device. The user can thus guide the applicator head precisely to the target object.

Further, according to a preferred embodiment of the present invention, the applicator head is configured to provide directionality of energy distribution, wherein the energy control unit is particularly configured to control the resulting spatial energy distribution in the target object as a function of at least one of: the relative pose of the head of the applicator with respect to the target object, the orientation of the head with respect to the target object, the position of the head with respect to the target object, medical image data, a property of the target object.

Furthermore, according to an embodiment, the head of the applicator comprises a plurality of independent energy emitting elements for delivering energy to the target object, wherein said elements are particularly arranged side by side along a longitudinal axis of the head and/or side by side along a periphery of the head (which periphery extends along a plane that runs perpendicular to said longitudinal axis). Particularly, the energy control unit is configured to control the respective energy emitting element so as to generate said resulting spatial energy distribution.

Further, according to a preferred embodiment of the present invention, the system comprises a plurality of applicators, wherein each applicator comprises a head and is configured to be inserted into the patient's body so as to position the respective head into or close to said target object (particularly a tumor) located inside said body, and wherein the respective applicator is configured to emit energy provided to the respective applicator via the respective head so as to create an ablation volume having a pre-defined shape and comprising at least a region of said target object. Furthermore, yet another aspect of the present disclosure relates to a method for controlling the amount of energy emitted per time by a head of an applicator for creating an ablation volume within a target object as claimed in claim 20.

According thereto, data relating to the relative pose of the head of the applicator with respect to the pose of the target object is measured by means of a tracking system upon guiding the applicator to the target object, and wherein the energy emitted by said head of the applicator is automatically controlled depending on said relative pose of the applicator with respect to the target object.

Further, according to a preferred embodiment of the present invention, both the pose of applicator's head and the pose of the target are measured by means of one of: electromagnetic, optical, optical-fiber-based or time-of-flight based tracking (see also above).

Further, according to a preferred embodiment of the present invention, the temporal and spatial delivery of energy via the head of the applicator is dynamically controlled based on an quasi-continuous input from the tracking system.

Further, according to a preferred embodiment of the present invention, energy (preferably in the form of alternating electromagnetic fields, e.g. radiofrequency, microwaves, light or other sorts of energy) is emitted via the applicator's head into the target object causing a tissue volume of interest near or around the target object or comprising the target object or at least portions thereof to be destroyed.

Further, according to a preferred embodiment of the present invention, the head of the applicator is guided manually to the target object.

Further, according to a preferred alternative embodiment of the present invention, the head of the applicator is automatically guided to the target object using a means for automatically moving the applicator, e.g. a robotic arm.

Further, according to a preferred embodiment of the present invention, said means is configured to move the head of the applicator upon emitting energy to the target object in a pre-defined way so as to give the ablation volume a pre-defined shape that is difficult or impossible to achieve manually otherwise.

Further, according to a preferred embodiment of the present invention, the position or pose of the target object is measured using an externally attached tracking device or using an internally placed tracking device (see also above), wherein particularly an internally placed tracking device (e.g. a beacon) may be placed using a needle like or catheter-like instrument near or within the target volume to be ablated as described above.

Further, according to a preferred embodiment of the present invention, both the pose of the head of the applicator and the pose of the target object are co-registered to a previously or instantaneously acquired medical image data set, allowing for subsequent annotations of anatomical structures and/or simulation and extrapolation of the ablation effects.

Further, according to a preferred embodiment of the present invention, the head of the applicator provides directionality of energy distribution, wherein particularly the resulting spatial energy distribution in the target object is controlled as a function of at least one of: the relative position of the applicator and the target object, available image data, and additionally available target properties.

Further, according to a preferred embodiment of the present invention, a plurality of applicators that may be designed as the applicator described herein, are used, wherein these applicators are introduced synchronously or asynchronously into or near the target object.

Further, according to a preferred embodiment of the present invention, energy is emitted into the target object via said plurality of individual applicators (e.g. via the respective head) so as to create a defined ablation volume according to a plan. Further, according to a preferred embodiment of the present invention, the creation of the pre-defined ablation volume is automatically controlled according to the pre-defined image-based plan.

Further features, embodiments, aspects and advantages of the present invention shall be described by means of a detailed description with reference to the Figures, wherein
- Fig.1: shows a schematic representation of a system according to the invention;
- Fig.2: shows the communication of individual components of the system according to the invention; and
- Fig.3: shows the possibility of a superposition of individual ablation volumes using the system according to the invention in order to create an overall ablation volume having a complex, pre-defined shape; and
- Figs. 4 to 6: show different heads of an applicator according to the present invention comprising a plurality of energy emitting elements, respectively.

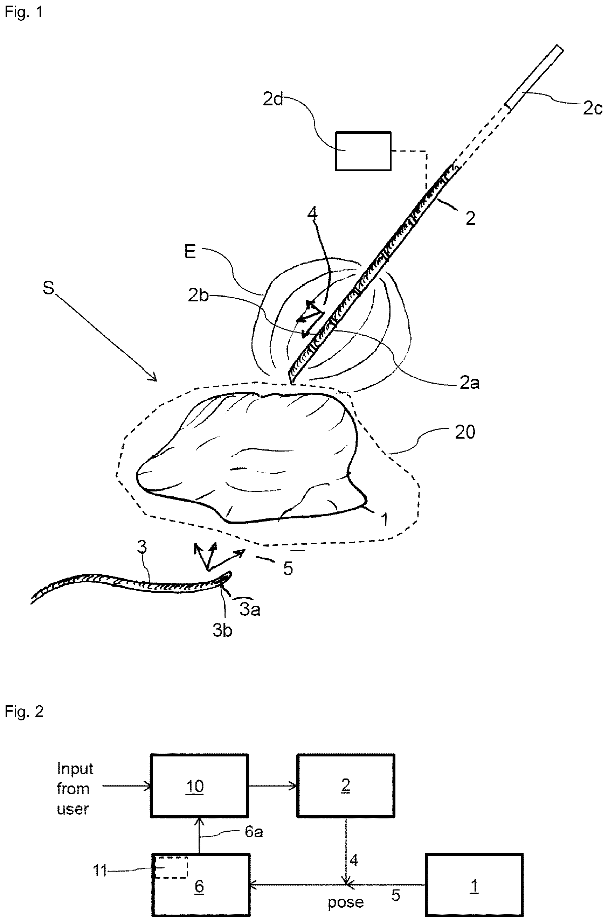

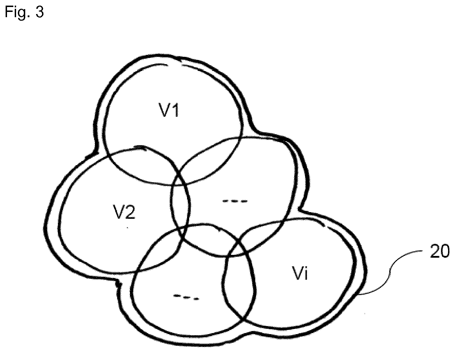

Fig. 1 shows in conjunction with Figs. 2 and 3 a system S for creating an ablation volume 20 within a target object 1 which may be a tumor located inside a patient's body that shall be destroyed by generating an ablation volume 20 that includes the target object 1, i.e., by heating the target object 1 up in a sufficient manner.

For this, the system S comprises an applicator 2, particularly in the form of an elongated needle that can be percutaneously brought into the vicinity of the target object 1. The applicator comprises a head 2a via which the applicator 2 is configured to emit energy E provided to the applicator 2 by means of a suitable energy source. Such applicators 2 are for instance described in [1].

This allows one to create an ablation volume 20 by controlling the amount of energy E emitted per time via said head 2a towards the target object 1.

The system further comprises a tracking system 6 configured to track the pose 4 of the head 2a with respect to the pose 5 of the target object 1, wherein the tracking system 6 is further configured to provide a signal 6a indicative of the relative pose of the head 2a of the applicator 2 with respect to the target object 1, and an energy control unit 10 configured to control emission of said energy E by the head 2a of the applicator 2 in response to said signal 6a. Particularly the energy control unit 10 is configured to automatically trigger emission of energy E via the head 2a of said applicator 2 towards the target object 1 for generating said ablation volume 20 when the distance between the head 2a and the target object 1 falls below a pre-defined value.

For proving data 6a concerning the spatial position of the head 2a of the applicator 2 with respect to the target object 1, the tracking system 6 may be configured to use a first tracking device 3b for measuring the pose 5 of the target object 1

Here, the system may comprises an elongated flexible means or device 3 (e.g. a catheter) comprising said first tracking device 3b at a tip 3a of said flexible means 3, wherein said flexible means 3 is configured to be placed into or close to the target object 1 via a body lumen of the patient. The first tracking device is preferably configured to transmit an electromagnetic signal which is indicative of the position of the tip 3a of the flexible means 3. Thus, by bringing the tip 3a of the flexible means 3 close to the target object 1, the first tracking device 3b actually reveals the position of the target object 1. A spatial offset between the target object 1 and said tip 3a may be determined by medical image data so that one can precisely determine the position of the target object once the position of the tip 3a is known.

The tracking system 6 may further comprise a second tracking device 2b which may also be formed as a (second) transmitter that is configured to transmit an electromagnet signal as well that is indicative of the position of the head 2a of the applicator 2. Thus, the tracking means/system may provide a signal 6a which provides the position of the head 2a of the applicator 2 with respect to the target object 1.

Particularly, said elongated flexible means/device 3 may be a catheter or a guide wire of a catheter, which guide wire may be preferably configured for transcatheter arterial chemoembolization (TACE). This allows one to combine the creation of an ablation volume 20 with a TACE treatment in an advantageous manner.

The energy control unit 10 is now configured to trigger emission of energy E via the head 2a towards the target object 1 in order to generate an ablation volume 20 comprising the target object 1, once the distance between the head 2a and the target object 1 falls below a certain threshold.

Using the signal 6a, the head 2a of the applicator 2 may also be automatically moved towards the target object 1 along a pre-definable track by means of a means 2d for automatically moving the applicator (such as a robot arm). This also allows one to create an ablation volume 20 of a more complex shape by generating smaller ablation volumes Vi at distinct locations as shown in Fig. 3. However, the applicator 2 may also be guided manually using e.g. a handle 2c of the applicator 2.

In order to be able to visually control and/or observe the ablation process, the tracking means 6 preferably comprises a display means 11 (e.g. a display for displaying information optically) for displaying e.g. the pose of the head 2a of the applicator 2 with respect to the pose of the target object 1.

Further, the complex ablation volume 20 shown in Fig. 3 may also be generated by using several applicators 2, wherein each applicator 2 comprises a head 2a and is configured to be inserted into the patient's body so as to position the respective head 2a into or close to said target object 1. Here, each applicator 2 may be configured to generate an ablation volume Vi which together form the ablation volume 20 shown in Fig. 3.

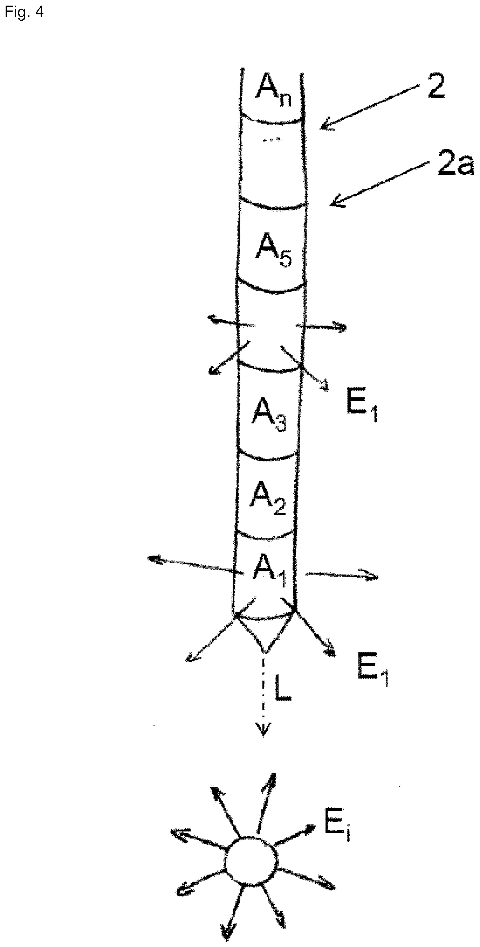

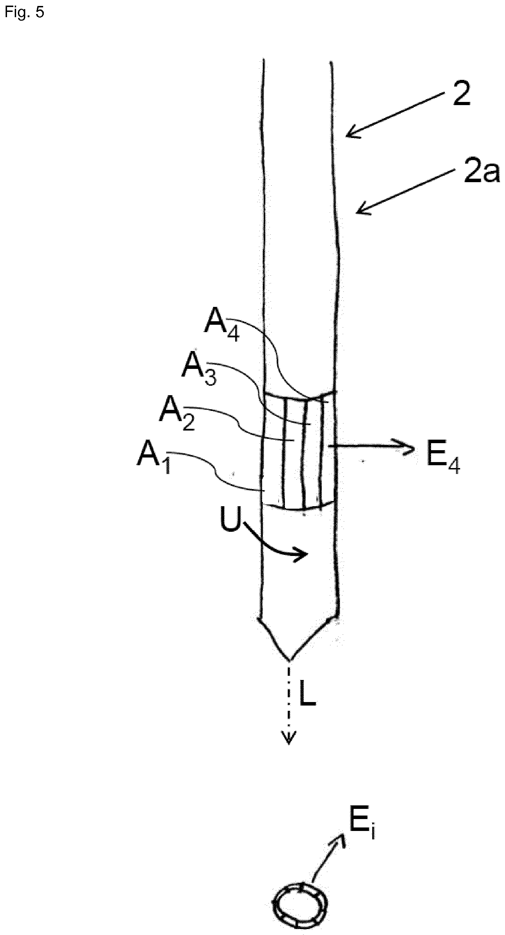

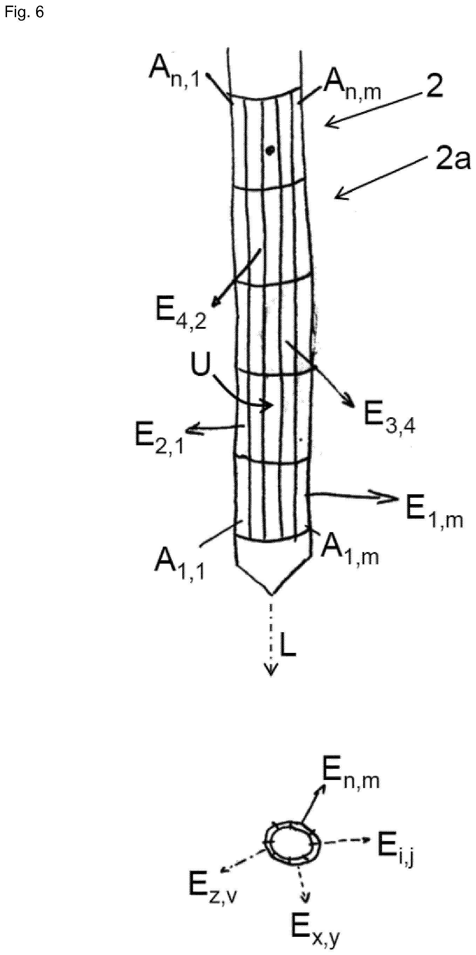

According to Figs. 4 to 6, the system according to the present invention can comprise a head 2a that comprises a plurality of independent energy emitting elements Aₙ or A_{n,m} for delivering energy Eᵢ or E_{i,j} to the target object 1 (not indicated in Figs. 4 to 6), wherein said elements Aₙ, A_{n,m} are arranged side by side along a longitudinal axis L of the respective head 2a and/or side by side along a periphery U of the respective head 2a, wherein particularly the energy control unit 10 is configured to control the respective energy emitting element Aₙ, A_{n,m} so as to generate a desired spatial energy distribution which can be different for different directions, depending on the control, arrangement and characteristics of the respective element Aₙ, A_{n,m}. Particularly, as shown in Fig. 4, several such elements A₁, A₂, ... ,Aₙ can be arranged one after the other along a longitudinal axis of the head 2a, wherein each element Aₙ is configured to independently emit energy Eᵢ in a cylindrical symmetrical fashion as indicated in the cross sectional view of Fig. 4 in the lower part of Fig. 4. Alternatively, as shown in Fig. 5, several such elements A₁, A₂, ... ,Aₙ can be arranged one after the other along the periphery U of the head 2a, wherein each element Aₙ is configured to independently emit energy Eᵢ in a certain direction (or predominantly in a certain direction, e.g. in a radial direction with respect to the head 2a) as indicated in the cross sectional view of Fig. 5 in the lower part of Fig. 5. Further, alternatively, as shown in Fig. 6, the embodiments of Figs. 4 and 5 may be combined, so that the head 2a comprises several such elements A_{n,m} which are arranged along the longitudinal axis L as well as along the periphery U. Thus, the head 2a comprises a two-dimensional array of such elements A_{n,m} arranged on the surface of the head 2a. Here, emission of energy can vary along the longitudinal axis L (as in Fig. 4) as well as in the direction of the periphery U (as in Fig. 5). Particularly, in an embodiment, the energy control unit 10 is configured to control the individual elements Aₙ or A_{n,m} depending on the relative pose of the head 2a (or of the individual element Aₙ or A_{n,m}) with respect to the target object 1. Thus, energy Eᵢ / E_{i,j} can be delivered to the target object 1 in a very precise manner.

### References

[1] Breen, D. J. & Lencioni, R. Nat. Rev. Clin. Oncol. 12, 175-186 (2015); published online 20 January 2015; doi:10.1038/nrclinonc.2014.237

## Claims

1. A system (S) for creating an ablation volume (20) within a target object (1), comprising:
- an applicator (2) comprising a head (2a), which applicator (2) is configured to be inserted into the patient's body so as to position said head (2a) into or close to a target object (1) located inside said body, and wherein the applicator (2) is configured to emit energy (E) provided to the applicator (2) via said head (2a) so as to create an ablation volume (20) comprising at least a region of said target object (1),
- a tracking system (6) configured to track the pose (4) of the head (2a) with respect to the pose (5) of the target object (1), wherein the tracking system (6) is further configured to provide a signal (6a) indicative of the relative pose of the head (2a) of the applicator (2) with respect to the target object (1), and
- an energy control unit (10) configured to control emission of said energy (E) per time by the head (2a) of the applicator (2) in response to said signal (6a).
- **characterized in that** the energy control unit (10) is configured to control emission of energy such that the energy that is delivered per unit time and unit of volume to the target object is a linear function derived from the relative pose between the head (2a) and the target object (1) or a derivative of said relative pose.

2. The system according to claim 1, **wherein** the energy control unit (10) is configured to automatically trigger emission of energy (E) via the head (2a) of said applicator (2) towards the target object (1) for generating said ablation volume (20) when the distance between the head (2a) and the target object (1) falls below a pre-defined value.

3. The system according to claim 1 or 2, **characterized in that** the tracking system (6) is configured to determine the pose (4) of the head (2a) of the applicator (2) and the pose of the target object (1) using one of: electromagnetic tracking, optical tracking, optical-fiber based tracking, time-of-flight-based tracking.

4. The system according to one of the preceding claims, **wherein** the tracking means (6) comprises a display means (11) for displaying the pose of the head (2a) of the applicator (2) with respect to the pose of the target object (1).

5. The system according to one of the preceding claims, **characterized in that** the energy control unit (10) is configured to control the temporal and spatial delivery of energy (E) to the target object (1) via the head (2a) of the applicator (2) using said signal (6a).

6. The system according to one of the preceding claims, **characterized in that** said delivered energy increases and/or decreases depending on the distance between the head (2a) and the target object (1) or depending on the position of the head (2a) with respect to the target object (1) or depending on the position of the head (2a) and on the spatial orientation of the head (2a) with respect to the target object (1).

7. The system according to one of the preceding claims, **characterized in that** said energy (E) which the applicator (2) is configured to emit via its head (2a) causes the target object (1) to heat up within said ablation volume so that subsequently material properties of the target object (1) in the ablation volume (20) are changed.

8. The system according to one of the preceding claims, **characterized in that** the applicator (2) comprises a handle (2c) for manually moving the applicator (2), or that the system (S) comprises a means (2d) or actuator (2d) for automatically moving the applicator (2).

9. The system according to claim 8, **characterized in that** the system (S) is configured to generate an ablation volume (20) of a pre-defined shape by moving the head (2a) of the applicator (2) along a pre-defined track by means of said means (2d) or actuator (2d) for automatically moving the applicator (2).

10. The system according to one of the preceding claims, **characterized in that** the tracking system (6) comprises a first tracking device (3b) for measuring the pose (5) of the target object (1), wherein said first tracking device (3b) is configured to be placed into the body of a patient, and wherein preferably the first tracking (3b) device is a first transmitter configured for transmitting an electromagnetic signal, or wherein said first tracking device is an externally attached first tracking device.

11. The system according to one of the preceding claims, **characterized in that** the tracking system (6) comprises a second tracking device (2b) for measuring the pose of the applicator head (2a), wherein said second tracking device (2b) is arranged on the head (2a) of the applicator (2), and wherein preferably the second tracking device (2b) is a second transmitter configured for transmitting an electromagnetic signal.

12. The system according to claim 10 or according to claim 11 when referring to claim 10, **characterized in that** the system (S) comprises an elongated flexible means (3) comprising said first tracking device (3b) at a tip (3a) of said flexible means (3), wherein said flexible means (3) is configured to be placed into or close to the target object (1) via a body lumen of the patient, wherein preferably said elongated flexible means (3) is a catheter or a guide wire (3) for a catheter

13. The system according to one of the preceding claims, **characterized in that** the head (2a) of the applicator (2) is configured to provide directionality of energy distribution, wherein particularly the energy control unit (10) is configured to control the resulting spatial energy distribution in the target object (1) as a function of at least one of: the relative pose of the head (2a) of the applicator (2) with respect to the target object (1), the orientation of the head (2a) with respect to the target object (1), the position of the head (2a) with respect to the target object (1), medical image data, a property of the target object (1).

14. The system according to one of the preceding claims, **characterized in that** the head of the applicator (2) comprises a plurality of independent energy emitting elements (Aₙ, A_{n,m}) for delivering energy to the target object (1), wherein said elements (Aₙ, A_{n,m}) are arranged side by side along a longitudinal axis (L) of the head (2a) and/or side by side along a periphery (U) of the head (2a), wherein particularly the energy control unit (10) is configured to control the respective energy emitting element (Aₙ, A_{n,m}) so as to generate said resulting spatial energy distribution.

15. The system according to one of the preceding claims, **characterized in that** the system (S) comprises a plurality of applicators (2), wherein each applicator (2) comprises a head (2a) and is configured to be inserted into the patient's body so as to position the respective head (2a) into or close to said target object (1) located inside said body, and wherein the respective applicator (2) is configured to emit energy (E) provided to the respective applicator (2) via the respective head (2a) so as to create an ablation volume (20, Vi) having a pre-defined shape and comprising at least a region of said target object (20).

## Patentansprüche

1. Ein System (S) zur Erzeugung eines Ablationsvolumens (20) innerhalb eines Zielobjekts (1), umfassend:
- einen Applikator (2), umfassend einen Kopf (2a), wobei der Applikator (2) dazu eingerichtet ist, in den Körper des Patienten eingeführt zu werden, um besagten Kopf (2a) in oder in der Nähe eines Zielobjekts (1) zu positionieren, das sich innerhalb besagten Körpers befindet, und wobei der Applikator (2) dazu eingerichtet ist, Energie (E) zu emittieren, die dem Applikator (2) über besagten Kopf (2a) bereitgestellt wird, um ein Ablationsvolumen (20) zu erzeugen, das mindestens einen Bereich besagten Zielobjekts (1) umfasst,
- ein Nachverfolgungssystem (6), das dazu eingerichtet ist, die Pose (4) des Kopfes (2a) in Bezug auf die Pose (5) des Zielobjekts (1) zu verfolgen, wobei das Nachverfolgungssystem (6) weiterhin dazu eingerichtet ist, ein Signal (6a) bereitzustellen, das indikativ für die relative Pose des Kopfes (2a) des Applikators (2) in Bezug auf das Zielobjekt (1) ist, und
- eine Energiekontrolleinheit (10), die dazu eingerichtet ist, die Abgabe der Energie (E) pro Zeit durch den Kopf (2a) des Applikators (2) in Reaktion auf besagtes Signal (6a) zu kontrollieren
**dadurch gekennzeichnet, dass**
die Energiekontrolleinheit (10) dazu eingerichtet ist, die Abgabe von Energie so zu kontrollieren, dass die pro Zeiteinheit und Volumeneinheit an das Zielobjekt abgegebene Energie eine lineare Funktion ist, die von der relativen Pose zwischen dem Kopf (2a) und dem Zielobjekt (1) oder einer Ableitung der besagter relativen Pose abgeleitet ist.

2. Das System gemäß Anspruch 1, **wobei** die Energiekontrolleinheit (10) dazu eingerichtet ist, automatisch die Emission von Energie (E) über den Kopf (2a) besagten Applikators (2) in Richtung des Zielobjekts (1) zur Generierung besagten Ablationsvolumens (20) auszulösen, wenn der Abstand zwischen dem Kopf (2a) und dem Zielobjekt (1) unter einen vordefinierten Wert fällt.

3. Das System gemäß Anspruch 1 oder 2, **dadurch gekennzeichnet, dass** das Nachverfolgungssystem (6) dazu eingerichtet ist, die Pose (4) des Kopfes (2a) des Applikators (2) und die Pose des Zielobjekts (1) unter Verwendung einer der folgenden Methoden zu bestimmen: elektromagnetische Nachverfolgung, optische Nachverfolgung, auf optischen Fasern basierende Nachverfolgung, auf Flugzeit basierende Nachverfolgung.

4. Das System gemäß einem der vorhergehenden Ansprüche, **wobei** die Nachverfolgungseinrichtung (6) ein Anzeigemittel (11) zur Anzeige der Pose des Kopfes (2a) des Applikators (2) in Bezug auf die Pose des Zielobjekts (1) umfasst.

5. Das System gemäß einem der vorhergehenden Ansprüche, **dadurch gekennzeichnet, dass** die Energiekontrolleinheit (10) dazu eingerichtet ist, die zeitliche und räumliche Abgabe von Energie (E) an das Zielobjekt (1) über den Kopf (2a) des Applikators (2) unter Verwendung besagten Signals (6a) zu kontrollieren.

6. Das System gemäß einem der vorhergehenden Ansprüche, **dadurch gekennzeichnet, dass** besagte abgegebene Energie in Abhängigkeit vom Abstand zwischen dem Kopf (2a) und dem Zielobjekt (1) oder in Abhängigkeit von der Position des Kopfes (2a) in Bezug auf das Zielobjekt (1) oder in Abhängigkeit von der Position des Kopfes (2a) und von der räumlichen Ausrichtung des Kopfes (2a) in Bezug auf das Zielobjekt (1) zunimmt und/oder abnimmt.

7. Das System gemäß einem der vorhergehenden Ansprüche, **dadurch gekennzeichnet, dass** besagte Energie (E), zu deren Emission über den Kopf (2a) der Applikator (2) eingerichtet ist, eine Erwärmung des Zielobjekts (1) innerhalb besagten Ablationsvolumens bewirkt, so dass anschließend Materialeigenschaften des Zielobjekts (1) im Ablationsvolumen (20) verändert werden.

8. Das System gemäß einem der vorhergehenden Ansprüche, **dadurch gekennzeichnet, dass** der Applikator (2) einen Griff (2c) zum manuellen Bewegen des Applikators (2) umfasst, oder dass das System (S) ein Mittel (2d) oder einen Aktuator (2d) zum automatischen Bewegen des Applikators (2) umfasst.

9. Das System gemäß Anspruch 8, **dadurch gekennzeichnet, dass** das System (S) dazu eingerichtet ist, ein Ablationsvolumen (20) einer vordefinierten Form durch Bewegen des Kopfes (2a) des Applikators (2) entlang einer vordefinierten Spur mittels des besagten Mittels (2d) oder Aktuators (2d) zum automatischen Bewegen des Applikators (2) zu erzeugen.

10. Das System gemäß einem der vorhergehenden Ansprüche, **dadurch gekennzeichnet, dass** das Nachverfolgungssystem (6) eine erste Nachverfolgungsvorrichtung (3b) zum Messen der Pose (5) des Zielobjekts (1) umfasst, wobei die erste Nachverfolgungsvorrichtung (3b) dazu eingerichtet ist, in den Körper eines Patienten platziert zu werden, und wobei vorzugsweise die erste Nachverfolgungsvorrichtung (3b) ein erster Transmitter ist, der zum Übertragen eines elektromagnetischen Signals eingerichtet ist, oder wobei besagte erste Nachverfolgungsvorrichtung eine extern angebrachte erste Nachverfolgungsvorrichtung ist.

11. Das System gemäß einem der vorhergehenden Ansprüche, **dadurch gekennzeichnet, dass** das Nachverfolgungssystem (6) eine zweite Nachverfolgungsvorrichtung (2b) zur Messung der Pose des Kopfes (2a) des Applikators (2) umfasst, wobei besagte zweite Nachverfolgungsvorrichtung (2b) auf dem Kopf (2a) des Applikators (2) angeordnet ist, und wobei vorzugsweise die zweite Nachverfolgungsvorrichtung (2b) ein zweiter Transmitter ist, der zur Übertragung eines elektromagnetischen Signals eingerichtet ist.

12. Das System gemäß Anspruch 10 oder gemäß Anspruch 11, wenn auf Anspruch 10 Bezug genommen wird, **dadurch gekennzeichnet, dass** das System (S) ein längliches flexibles Mittel (3) umfasst, das die erste Nachverfolgungsvorrichtung (3b) an einer Spitze (3a) besagten flexiblen Mittels (3) aufweist, wobei besagtes flexibles Mittel (3) dazu eingerichtet ist, über ein Körperlumen des Patienten in oder nahe dem Zielobjekt (1) platziert zu werden, wobei vorzugsweise besagtes längliches flexibles Mittel (3) ein Katheter oder ein Führungsdraht (3) für einen Katheter ist.

13. Das System gemäß einem der vorhergehenden Ansprüche, **dadurch gekennzeichnet, dass** der Kopf (2a) des Applikators (2) dazu eingerichtet ist, eine Richtungsabhängigkeit der Energieverteilung bereitzustellen, wobei insbesondere die Energiekontrolleinheit (10) dazu eingerichtet ist, die resultierende räumliche Energieverteilung in dem Zielobjekt (1) zu kontrollieren, als eine Funktion von mindestens einem von: die relative Pose des Kopfes (2a) des Applikators (2) in Bezug auf das Zielobjekt (1), die Ausrichtung des Kopfes (2a) in Bezug auf das Zielobjekt (1), die Position des Kopfes (2a) in Bezug auf das Zielobjekt (1), medizinische Bilddaten, eine Eigenschaft des Zielobjekts (1).

14. Das System gemäß einem der vorhergehenden Ansprüche, **dadurch gekennzeichnet, dass** der Kopf des Applikators (2) eine Vielzahl von unabhängigen Energieemissionselementen (Aₙ, A_{n,m}) zur Zufuhr von Energie zu dem Zielobjekt (1) umfasst, wobei besagte Elemente (Aₙ, Aₙ,ₘ) nebeneinander entlang einer Längsachse (L) des Kopfes (2a) und/oder nebeneinander entlang einer Peripherie (U) des Kopfes (2a) angeordnet sind, wobei insbesondere die Energiekontrolleinheit (10) dazu eingerichtet ist, das jeweilige Energieemissionselement (Aₙ, A_{n,m}) zu kontrollieren, um besagte resultierende räumliche Energieverteilung zu erzeugen.

15. Das System gemäß einem der vorhergehenden Ansprüche, **dadurch gekennzeichnet, dass** das System (S) eine Vielzahl von Applikatoren (2) umfasst, wobei jeder Applikator (2) einen Kopf (2a) umfasst und dazu eingerichtet ist, in den Körper des Patienten eingeführt zu werden, um den jeweiligen Kopf (2a) in oder nahe dem Zielobjekt (1) zu positionieren, das sich innerhalb des Körpers befindet, und wobei der jeweilige Applikator (2) dazu eingerichtet ist, Energie (E) zu emittieren, die dem jeweiligen Applikator (2) über den jeweiligen Kopf (2a) bereitgestellt wird, um ein Ablationsvolumen (20, Vi) zu erzeugen, das eine vordefinierte Form aufweist und mindestens einen Bereich besagten Zielobjekts (20) umfasst.

## Revendications

1. Système (S) pour créer un volume d'ablation (20) au sein d'un objet cible (1), comprenant :
- un applicateur (2) comprenant une tête (2a), lequel applicateur (2) est configuré pour être inséré dans le corps du patient de manière à positionner ladite tête (2a) dans ou près d'un objet cible (1) situé à l'intérieur dudit corps, et dans lequel l'applicateur (2) est configuré pour émettre de l'énergie (E) fournie à l'applicateur (2) par le biais de ladite tête (2a) de manière à créer un volume d'ablation (20) comprenant au moins une région dudit objet cible (1),
- un système de traçage (6) configuré pour tracer la pose (4) de la tête (2a) par rapport à la pose (5) de l'objet cible (1), dans lequel le système de traçage (6) est en outre configuré pour fournir un signal (6a) indicateur de la pose relative de la tête (2a) de l'applicateur (2) par rapport à l'objet cible (1), et
- un module de commande d'énergie (10) configuré pour commander l'émission de ladite énergie (E) par temps par la tête (2a) de l'applicateur (2) en réponse audit signal (6a),
- **caractérisé en ce que** le module de commande d'énergie (10) est configuré pour commander l'émission d'énergie de sorte que l'énergie qui est fournie par temps unitaire et unité de volume à l'objet cible est une fonction linéaire dérivée de la pose relative entre la tête (2a) et l'objet cible (1) ou une dérivée de ladite pose relative.

2. Système selon la revendication 1, **dans lequel** le module de commande d'énergie (10) est configuré pour déclencher automatiquement l'émission d'énergie (E) par le biais de la tête (2a) dudit applicateur (2) vers l'objet cible (1) pour générer ledit volume d'ablation (20) lorsque la distance entre la tête (2a) et l'objet cible (1) tombe en dessous d'une valeur prédéfinie.

3. Système selon la revendication 1 ou 2, **caractérisé en ce que** le système de traçage (6) est configuré pour déterminer la pose (4) de la tête (2a) de l'applicateur (2) et la pose de l'objet cible (1) en utilisant un traçage parmi : le traçage électromagnétique, le traçage optique, le traçage à base de fibre optique, le traçage à base du temps de vol.

4. Système selon une des revendications précédentes, **dans lequel** le moyen de traçage (6) comprend un moyen d'affichage (11) pour afficher la pose de la tête (2a) de l'applicateur (2) par rapport à la pose de l'objet cible (1).

5. Système selon une des revendications précédentes, **caractérisé en ce que** le module de commande d'énergie (10) est configuré pour commander la fourniture temporelle et spatiale d'énergie (E) à l'objet cible (1) par le biais de la tête (2a) de l'applicateur (2) en utilisant ledit signal (6a).

6. Système selon une des revendications précédentes, **caractérisé en ce que** ladite énergie fournie augmente et/ou diminue en fonction de la distance entre la tête (2a) et l'objet cible (1) ou en fonction de la position de la tête (2a) par rapport à l'objet cible (1) ou en fonction de la position de la tête (2a) et de l'orientation spatiale de la tête (2a) par rapport à l'objet cible (1).

7. Système selon une des revendications précédentes, **caractérisé en ce que** ladite énergie (E) que l'applicateur (2) est configuré pour émettre par le biais de sa tête (2a) provoque le chauffage de l'objet cible (1) au sein dudit volume d'ablation de sorte que des propriétés matérielles de l'objet cible (1) dans le volume d'ablation (20) sont ensuite changées.

8. Système selon une des revendications précédentes, **caractérisé en ce que** l'applicateur (2) comprend une poignée (2c) pour déplacer manuellement l'applicateur (2), ou que le système (S) comprend un moyen (2d) ou actionneur (2d) pour déplacer automatiquement l'applicateur (2).

9. Système selon la revendication 8, **caractérisé en ce que** le système (S) est configuré pour générer un volume d'ablation (20) d'une forme prédéfinie en déplaçant la tête (2a) de l'applicateur (2) le long d'une piste prédéfinie au moyen dudit moyen (2d) ou actionneur (2d) pour déplacer automatiquement l'applicateur (2).

10. Système selon une des revendications précédentes, **caractérisé en ce que** le système de traçage (6) comprend un premier dispositif de traçage (3b) pour mesurer la pose (5) de l'objet cible (1), dans lequel ledit premier dispositif de traçage (3b) est configuré pour être placé dans le corps d'un patient, et dans lequel le premier dispositif de traçage (3b) est de préférence un premier émetteur configuré pour transmettre un signal électromagnétique, ou dans lequel ledit premier dispositif de traçage est un premier dispositif de traçage relié de manière externe.

11. Système selon une des revendications précédentes, **caractérisé en ce que** le système de traçage (6) comprend un second dispositif de traçage (2b) pour mesurer la pose de la tête d'applicateur (2a), dans lequel ledit second dispositif de traçage (2b) est agencé sur la tête (2a) de l'applicateur (2), et dans lequel le second dispositif de traçage (2b) est de préférence un second émetteur configuré pour transmettre un signal électromagnétique.

12. Système selon la revendication 10 ou selon la revendication 11 lorsqu'elle se réfère à la revendication 10, **caractérisé en ce que** le système (S) comprend un moyen flexible allongé (3) comprenant ledit premier dispositif de traçage (3b) à une pointe (3a) dudit moyen flexible (3), dans lequel ledit moyen flexible (3) est configuré pour être placé dans ou près de l'objet cible (1) par le biais d'une lumière corporelle du patient, dans lequel ledit moyen flexible allongé (3) est de préférence un cathéter ou un fil-guide (3) pour un cathéter.

13. Système selon une des revendications précédentes, **caractérisé en ce que** la tête (2a) de l'applicateur (2) est configurée pour fournir une directionnalité de distribution d'énergie, dans lequel le module de commande d'énergie (10) est notamment configuré pour commander la distribution d'énergie spatiale résultante dans l'objet cible (1) en tant que fonction d'au moins un élément parmi : la pose relative de la tête (2a) de l'applicateur (2) par rapport à l'objet cible (1), l'orientation de la tête (2a) par rapport à l'objet cible (1), la position de la tête (2a) par rapport à l'objet cible (1), des données d'image médicale, une propriété de l'objet cible (1).

14. Système selon une des revendications précédentes, **caractérisé en ce que** la tête de l'applicateur (2) comprend une pluralité d'éléments émetteurs d'énergie indépendants (Aₙ, A_{n,m}) pour fournir de l'énergie à l'objet cible (1), dans lequel lesdits éléments (Aₙ, A_{n,m}) sont agencés côte à côte le long d'un axe longitudinal (L) de la tête (2a) et/ou côte à côte le long d'une périphérie (U) de la tête (2a), dans lequel le module de commande d'énergie (10) est notamment configuré pour commander l'élément émetteur d'énergie respectif (Aₙ, A_{n,m}) de manière à générer ladite distribution d'énergie spatiale résultante.

15. Système selon une des revendications précédentes, **caractérisé en ce que** le système (S) comprend une pluralité d'applicateurs (2), dans lequel chaque applicateur (2) comprend une tête (2a) et est configuré pour être inséré dans le corps du patient de manière à positionner la tête respective (2a) dans ou près dudit objet cible (1) situé à l'intérieur dudit corps, et dans lequel l'applicateur respectif (2) est configuré pour émettre de l'énergie (E) fournie à l'applicateur respectif (2) par le biais de la tête respective (2a) de manière à créer un volume d'ablation (20, Vi) ayant une forme prédéfinie et comprenant au moins une région dudit objet cible (20).
